# EUROPEAN PATENT SPECIFICATION

(11) **EP 3 592 408 B1**
(45) Date of publication and mention of the grant of the patent: **23.02.2022**
(21) Application number: 18719643.1
(22) Date of filing: 01.03.2018
(51) Int. Cl.: A61M 5/32, A61M 25/00, A61M 25/06, A61M 5/31, A61M 39/06, F16K 5/00, A61M 39/22, A61M 39/24

(54) **IMPROVED SAFETY CATHETER NEEDLE**
VERBESSERTE SICHERHEITSKATHETERNADEL
AIGUILLE DE CATHÉTER DE SÉCURITÉ AMÉLIORÉE

(30) Priority: 07.03.2017 IT 201700025175
(43) Date of publication of application: 15.01.2020
(73) Proprietor: Sol-Millennium Swiss R&D Center SA, 6900 Lugano (CH)
(72) Inventor: DE ZOLT, Dario, 21054 Fagnano Olona (VA) (IT); LAGANA', Matteo, 22030 Longone al Segrino (CO) (IT)
(74) Representative: Ripamonti, Enrico
(86) International application number: PCT/IB2018/051309
(87) International publication number: WO 2018/163028

(56) References cited:
- EP-A1- 0 645 159
- EP-A1- 1 568 393
- WO-A2-2008/008206

## Description

The present invention relates to a safety catheter needle according to the precharacterising clause of the principal claim.

As is known, a catheter needle or merely "catheter" is a medical device which enables a catheter of plastics material to be inserted into the peripheral venous system of a patient or into a vein of the latter or into the subcutaneous adipose tissue of the patient in order to be able to administer or extract liquids to or from the latter's body. For the catheter to be inserted into the patient's body provision is made for a metal cannula (with a cutting end projecting from the catheter) inserted within the catheter itself which has the function of inserting the latter into for example a vein of the patient and which is removed by a health professional who performs this insertion operation at the end of the catheter positioning procedure.

With a venous catheter of the type mentioned above there is the problem that when the metal cannula is extracted from the then positioned catheter the health professional may be accidentally pierced by the cannula; this may happen when the metal cannula is disposed of. This may give rise to problems in that the metal cannula has been contaminated with the patient's body fluids and may be a vehicle for the transmission of infectious diseases.

It is therefore of fundamental importance to provide catheters equipped with safety systems which limit if they do not completely rule out this risk.

Catheters provided with incorporated safety devices protecting health professionals from the risk of accidental punctures and contamination with patients' blood are known on the market. As mentioned, this is with the object of limiting the possibility of contaminating health professionals with patients' biological fluids. These catheters provided with safety devices (which we will refer to as "safety catheters") make it possible to reduce the risk of transmitting infectious diseases which can be carried by blood, such as AIDS, viral hepatitis and the like.

Known safety catheters have incorporated safety devices having different functions and configurations. Some of these, for example, only protect the tip of the cannula once the latter has been extracted from the catheter, this protection being obtained by enveloping the tip of the cannula in bodies associated with the catheter. See for example the following patents: WO2013021350, GB2451153B, WO2008014908, WO2016077234, GB2535671A, US5344408, US8591467, US2014018738, EP1114652B1, US7530965, US20040225260, EP2077133, EP2161049, WO2013187827, US20090216201, US5419766, US7887516.

Devices capable of protecting both the tip and the surface of the cannula by means of telescopic systems or sliding shells activated manually by the health professional after the cannula has been extracted from the catheter are in particular known; such are for example described in US4747831 and US6436070. These devices are referred to as safety catheters of the active type.

Other devices which operate in passive mode which snap to resiliently immobilise the cannula within the protective body, as described in US2016220791, are known. However, it is also known that systems provided with resilient elements immobilising the cannula may have disadvantages because of the excessive speed with which the cannula is retracted, giving rise for example to small amounts body fluids being splashed outside the body containing the tip, a high noise level and vibration of the resilient member immobilising the tip at the end of its travel.

The systems for enveloping only the tip after the cannula has been extracted from the catheter may be either housed within a catheter holder or outside it. When removed outside the catheter holder the systems protecting only the tip often have lateral or frontal openings such that in principle they do not prevent small drops of patients' body fluid from emerging, and this might give rise to problems for the health professional.

In addition to this, if the cannula is deformed, it is not possible to rule out a priori the possibility that the contaminated tip may come in contact with the health professional through openings present in the protecting body.

Safety catheters provided with devices capable of preventing a health professional from coming into contact with the tip of the cannula after the catheter has been extracted and which operate using a magnetic force capable of closing off access by the health professional to a body in which the aforesaid tip has been withdrawn after extraction of the catheter are known. For example, EP1568393 describes a protection system outside the catheter holder comprising a body for containing the tip of the cannula after use; this body has a moving element which can close off a passage through which the tip of the cannula returns within the containment body, said passage being specific to that body. In particular a kind of shutter (the moving element) which is drawn back magnetically from a fixed body is provided, this withdrawal action intercepting the aforesaid opening after the tip of the cannula has passed through so as to prevent it from projecting from said containment body. The moving element and the fixed body may be two magnetic bodies. These bodies are separate from each other and relatively distant from each other.

This solution is relatively difficult to implement because it requires bodies attracting each other magnetically which are of very small size and are difficult to insert into the containment body have to be produced, and the movement of one of such bodies does not have to be limited in any way for the opening in the protective body to be effectively closed.

In particular the moving element and the magnetic fixed body have to be at a very short distance from each other in order to be able to operate appropriately. This places a constraint on manufacture of the parts (at least the moving element) with very tight construction tolerances, something which is in itself very difficult. For this very reason even small misalignments between the fixed body and the moving element or even small variations in construction may result in incorrect movement of the moving element with consequent malfunction of the protective system described above.

EP2037989 (which derives from PCT patent application no. WO2008/008206 and which constitutes the precharacterising clause of the independent claim) describes a safety catheter needle comprising a cannula, a cannula holder and a safety device surrounding a portion of the cannula; this safety device comprises a safety body or container (safety housing) containing at least one magnet (a permanent magnet or an electromagnet powered by a battery) which is capable of causing an element blocking an opening in such body through which the tip of the cannula passes after the latter has been extracted from the catheter to rotate in order to retain such tip within said body. The magnet and the blocking element are two separate bodies and are completely independent from each other from the construction point of view.

The blocking element, which is constructed of magnetically sensitive material (for example ferromagnetic material) is located transversely to the axis of the cannula and comprises an opening through which the cannula moves, a transverse barrier (at right angles to the cannula and in which the opening is made) and a sensitive terminal member. The blocking element is attracted by the magnet which is well separated from that element.

More particularly, the magnet is attached to the safety container in such a way that the blocking element is attracted to it. This magnet may attract the transverse barrier or the terminal member, depending upon the position of the magnet with respect to the blocking element. Mutual attraction brings about rotation of the blocking element within the safety container: this blocking element usually bears on the cannula when the latter is in the position of use in which it projects from the safety container. After use the cannula is withdrawn towards the safety container in such a way that its distal extremity can be immobilised within the safety container. During this movement the cannula slides relative to the terminal body of the blocking element which bears against the cannula.

When the distal extremity penetrates the safety container beyond the aforesaid terminal body, the magnetic force between the magnet and the blocking element causes such blocking element to rotate within the safety container and the terminal body is positioned in the container in such a way as to prevent the distal extremity of the cannula from again emerging from the safety container.

This solution is also difficult to implement because of the dimensions of the magnet located within such body, which makes it difficult to attach it to a wall of the body or safety container and may give rise to problems in use because of the release of body fluid within the aforesaid body transported by the cannula.

Furthermore, as in the case of EP1568393, even the safety catheter needle described EP2037989 requires the magnetic element and the blocking element acting together therewith (separate and independent of each other) to be located at a very small distance from each other so that the magnetic force between them has the highest possible value to ensure that the blocking element moves.

Positioning of said individual elements in the known safety device is therefore of absolute importance in EP2037989, and this constrains the manufacturer to produce components with the smallest possible tolerances and the maximum accuracy in positioning.

In addition to this, in EP2037989 the blocking element can be moved in any way, after it has been carried frontally to the tip of the cannula, because of a thrust from the cannula itself onto such element. This may give rise to the possibility of such tip projecting from the safety device, with the risks mentioned above to health professionals.

Again, with regard to manufacture of the individual components of the invention which is the subject matter of EP2037989, the difficulty of assembling these components, including in relation to the means which enable the blocking element to move, must also be taken into consideration.

From all this it can be deduced that the invention which is the subject matter of EP2037989 is extremely difficult to implement, and in any event costly, while not ensuring total safety from the health professional's point of view.

EP0645159 describes a catheter with a protection element. The latter comprises a flap clip which when the cannula is outside the protective element bears against the surface of the cannula and moves resiliently within the protection element when the tip of the cannula is withdrawn from such protection element. Through this resilient movement the metal flap clip moves between the tip of the cannula and the opening of the protection element through which the cannula slides, immobilising the tip of the latter within the protective element.

This prior document does not describe that the flap clip is moved by means of a magnetic force, but resilient movement of the clip after the tip of the cannula has returned within the protective element is described.

The object of the present invention is to provide a safety catheter needle (or merely "safety catheter") of the abovementioned type which is improved in comparison with similar known solutions.

In particular the object of the present invention is that of providing a catheter which offers improved safety not only with respect to the fact that the tip of the cannula cannot come into contact with the health professional, but also in that there is no possibility that body fluids may emerge from the protective element and come into contact with the health professional.

Another object of the present invention is that of providing a safety catheter of the type mentioned which operates in wholly passive mode or without the health professional having to act in order to achieve protection of the tip of the cannula by acting on the latter or on members associated with it.

Another object is that of providing a safety catheter of the type mentioned in which the tip of the cannula is absolutely inaccessible to an operator after it has been extracted from the catheter holder.

Another object is to provide a safety catheter of the abovementioned type which does not lose its protection characteristics over time, said characteristics remaining unchanged even after different periods of time after production of the catheter.

Another object is that of providing a safety catheter which has a reduced risk of malfunction or which operates safely so as to ensure effective safety for health professionals.

Another object is that of providing a safety catheter which has a small number of components and is simple to manufacture and assemble, such as to have high reliability in use and low cost; this in particular with reference to its components which prevent contact between the tip of the cannula after the catheter has been extracted.

These and other objects which will be apparent to those skilled in the art will be accomplished through a venous catheter according to claim 1.

For a better understanding of the present invention there are attached purely by way of example and without limitation the following drawings, in which:
Figure 1 shows a perspective view of a safety catheter according to the present invention;
Figure 2 shows an exploded perspective view of the catheter in Figure 1 in which all the components of the safety catheter are shown;
Figure 3 shows a cross-section along the line 3-3 in Figure 1;
Figure 4 shows an exploded view of a protection device of the safety catheter according to the invention;
Figure 5 shows an assembled perspective view of the protection device in Figure 4;
Figures 6A and 6B show cross-sections along the line 6-6 in Figure 5 with the protection device in two operating positions;
Figures from 7A to 7C show various stages in use of the safety catheter according to the invention;
Figures 8A and 8B show in magnified view the parts indicated by A respectively in Figures 7B and 7C;
Figure 9 shows a perspective view of a variant of a catheter according to the invention;
Figure 10 shows a perspective view in longitudinal cross-section of the catheter in Figure 9;
Figure 11 shows a magnified view of the detail indicated by W in Figure 10;
Figures 12A, 12B, 12C show three different stages in use of the catheter in Figure 9;
Figure 13 shows a perspective view of a detail of a further embodiment of the invention; and
Figures 14 and 15 show views of details of the catheter in longitudinal cross-section during two different stages of use.

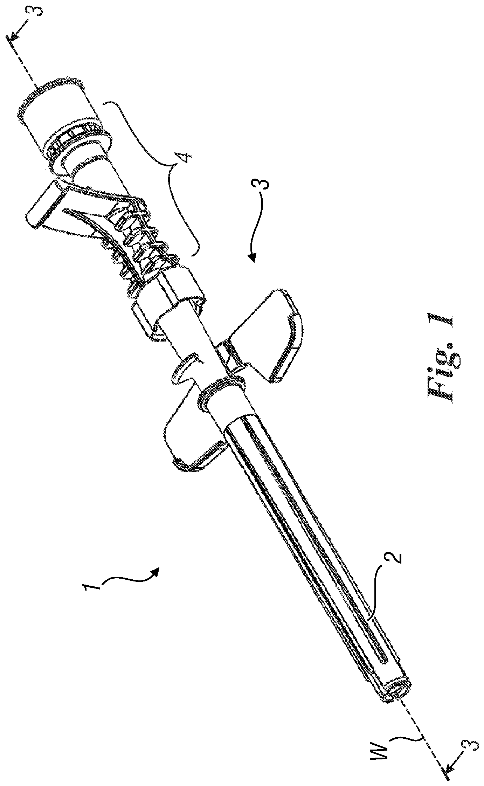

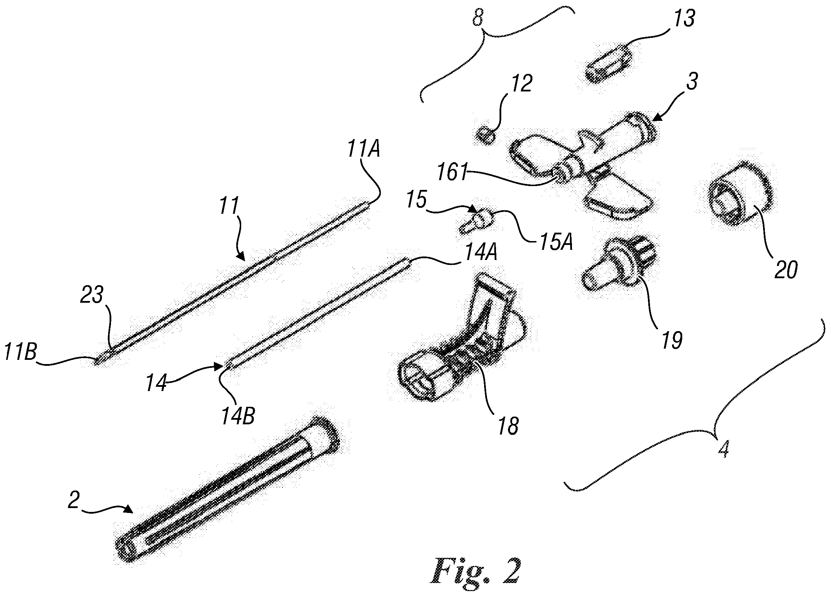

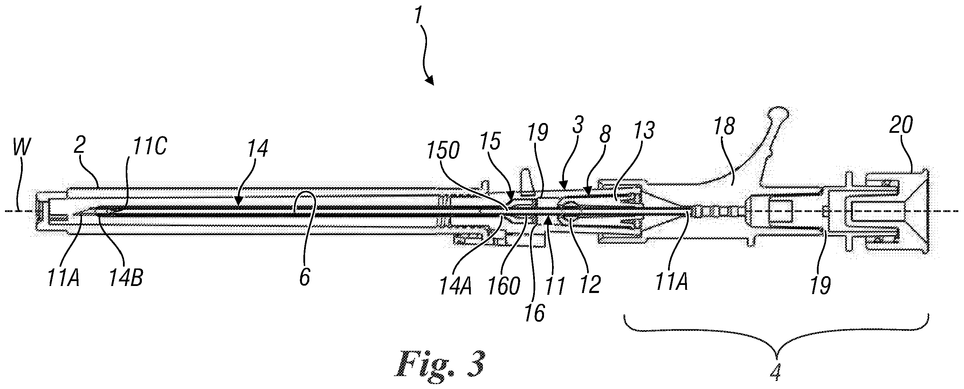

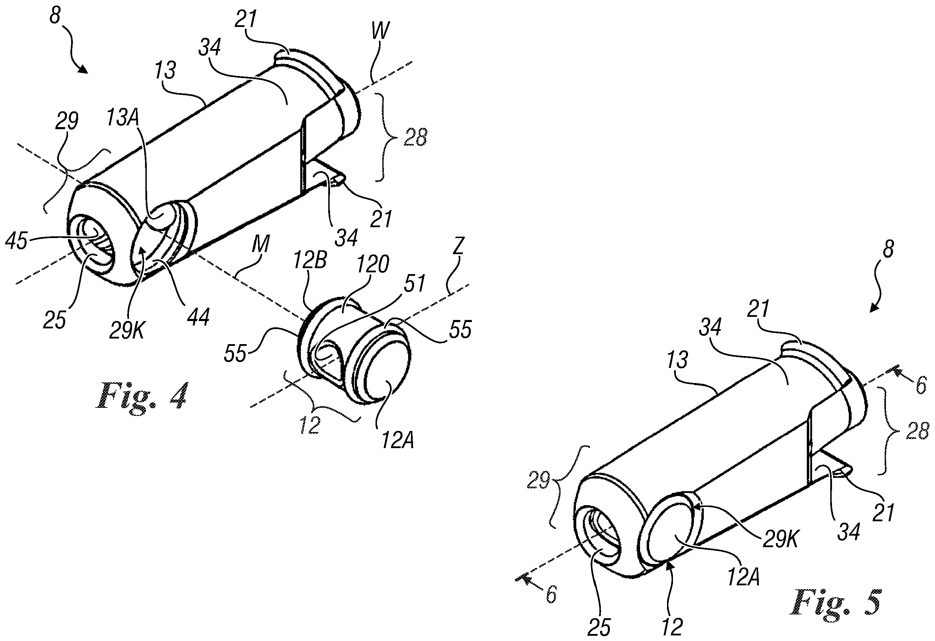

With reference to Figures 1-8, these show a safety catheter needle (or "safety catheter") according to the invention; the catheter is indicated as a whole by the reference number 1. Such a safety catheter comprises as a whole (see Figure 1, for example) a protection element or protection cover 2 which when associated with safety catheter 1 is associated in a known way with a catheter holder 3 which in turn is removably attached to a cannula holder 4. In particular the cannula holder comprises three components which are in themselves known (indicated by 18, 19 and 20 in the figures) which are capable of supporting a cannula 11 having a proximal extremity 11A of one piece with cannula holder 4 and a distal extremity 11B. Cannula 11 has a variable cross-section, for example a bevel 11C close to distal extremity 11B.

The reference to proximal and distal relates to the positions of cannula 11 with respect to cannula holder 4; all further indications of "proximal" or "distal" even when referring to other components of safety catheter 1 will refer to the latter.

Metal cannula 11 has a distal extremity 11B (or tip of the cannula) constructed so as to be cutting, for example through a cut along a plane inclined with respect to longitudinal axis W of the cannula (and entire safety catheter 1). This cannula 11 is capable of permitting a catheter 14 to be inserted into a vein or into the adipose tissue of a patient, an operation which is normally performed by a health professional. For this purpose, catheter 14 has a longitudinal through hole 6; a distal extremity or tip 11B of the cannula projects from a distal extremity of catheter 14B before and during the aforesaid operation.

Catheter 14 has a proximal extremity 14A which is of one piece with a body 15 (in itself known), with a through hole 150 positioned and immobilised between catheter holder 3 in a distal portion 160 of a cavity 16 of such catheter holder 3. In order to immobilise said body in said portion 160, in a manner which is in itself known, the latter is bounded by a first internal collar 19 of cavity 16 which abuts against a proximal extremity 15A of body 15 (through the hole of which cannula 11 passes) which is thus completely contained within portion 160 between collar 19 and distal extremity 161 of cavity 16 in catheter holder 3.

In the embodiment in the figures in question a protection and safety device 8 capable of receiving and retaining therein the distal extremity 11B of cannula 11 after catheter 14 has been inserted into a patient's body and such cannula has been extracted from the catheter is inserted and immobilised in cavity 16 of the catheter holder.

More particularly, safety device 8 is of the passive type and comprises a containment body 13 (or "first body") having a substantially cylindrical shape and having a through longitudinal cavity 13A (arranged, like all of body 13, along the W axis); this cavity has a proximal opening 24 in a proximal portion 28 of body 13 and a distal opening 25 in a distal portion 29 of that body (which also represents the distal extremity of protection device 8). This containment body 13 is capable of containing the distal extremity or tip 11B of cannula 11 after the cannula has been extracted from catheter 14 in order to protect a health professional who has performed the abovementioned extraction operation.

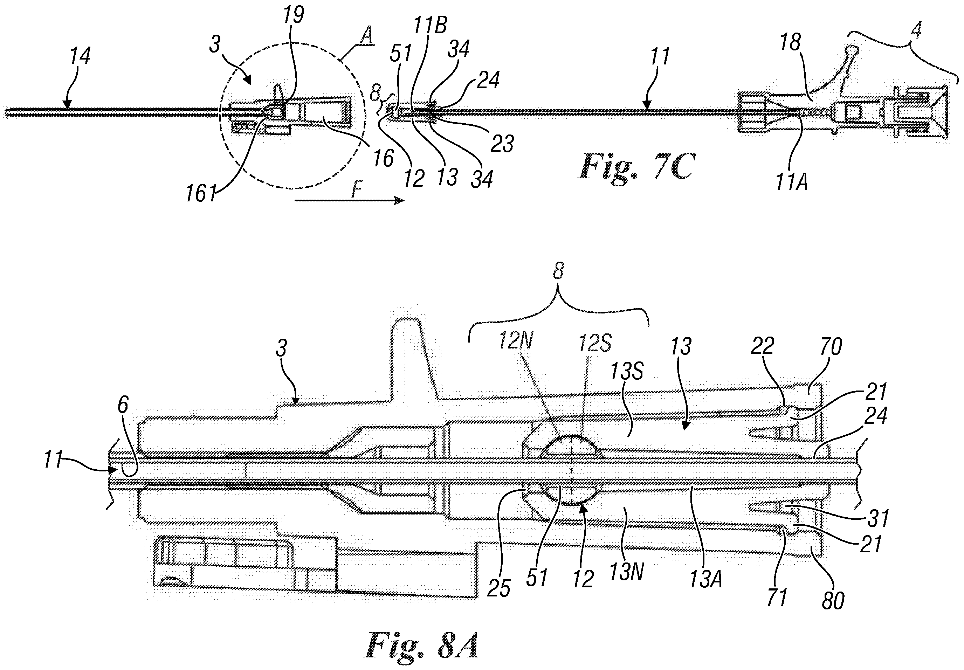

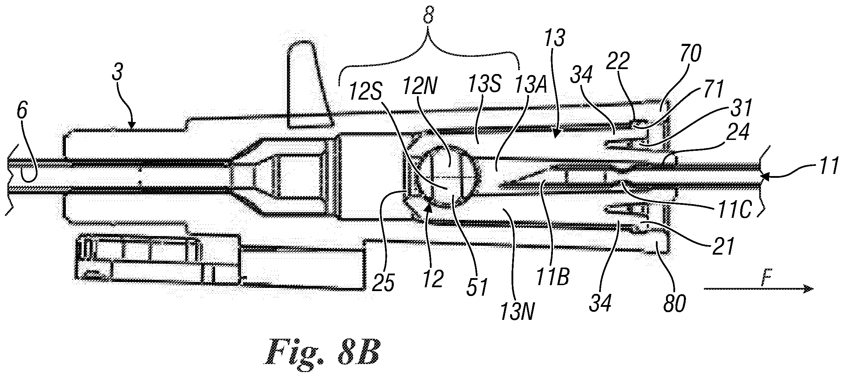

Containment body 13, at least partly corresponding to distal opening 25, is manufactured as a magnet having a first part 13N of positive (or N) polarity and a second part 13S (or S) of negative polarity. As an example, in the figures (as indicated in particular in Figures 8A and 8B), the whole of body 13 is constructed as a magnet and in the figures mentioned the first part 13N is located below second part 13S. This is obviously not limiting.

Containment body 13 also has an internal collar 31 close to proximal opening 24. In addition to this, by way of an example of an embodiment which is not limiting, this body 13 has two arms 34 (which may also be present in greater number) in proximal portion 28, each of which has a projecting flange 21 capable of acting together with abovementioned collar 31. Each of these arms is capable of acting together with a collar 80 provided between cavity 16 of catheter holder 3 close to a proximal extremity 70 thereof with a recess 71 close to such collar.

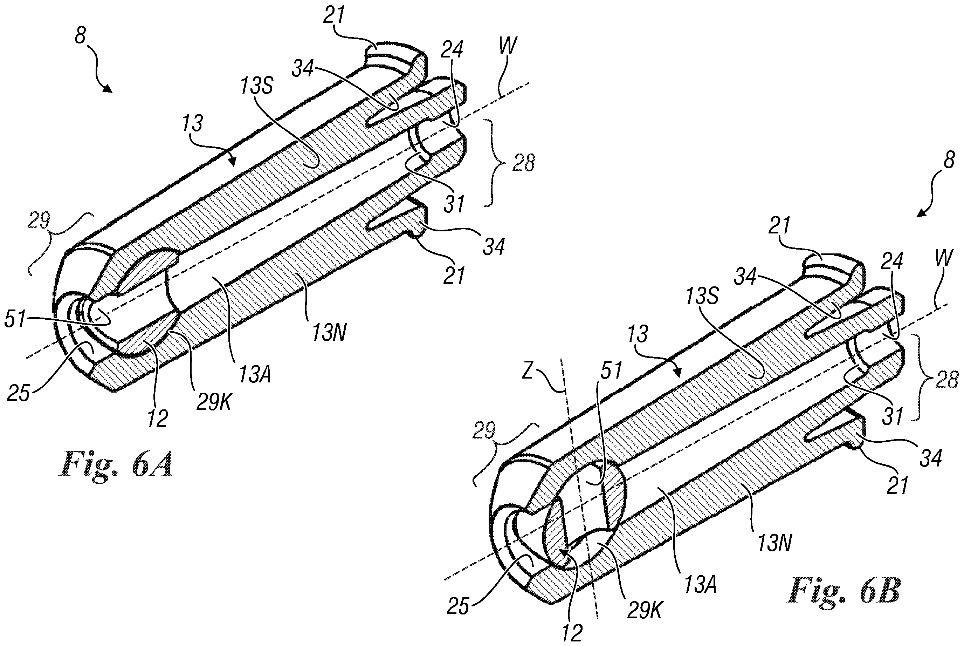

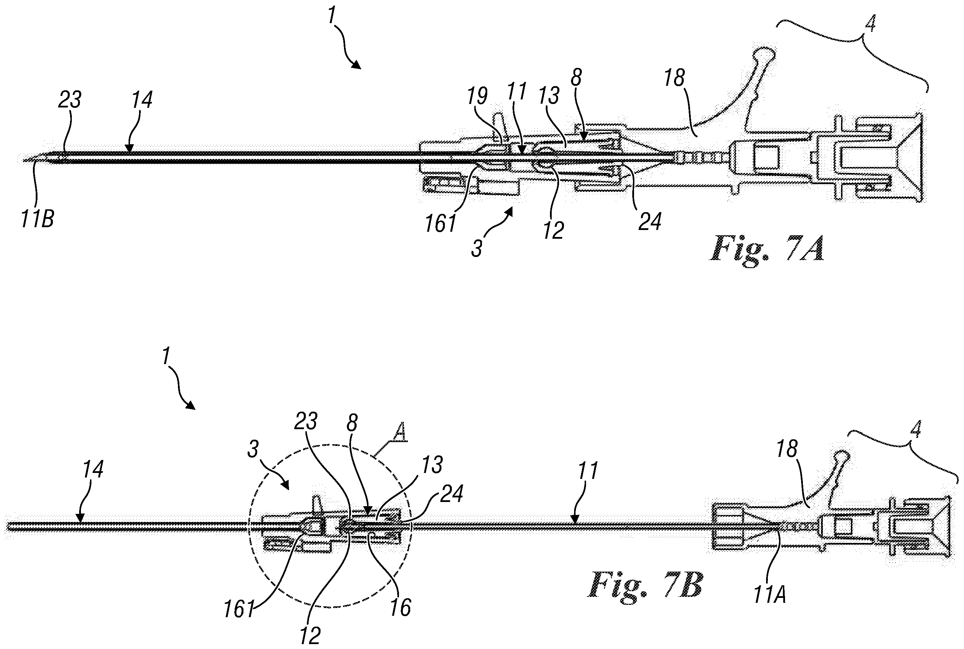

More particularly, in the (non-limiting) example in the figures, flange 21 has an external shape such that it can slide on collar 80 if containment body 13 experiences traction in a direction such as to extract it from catheter holder 3 in a direction opposite to that acting on catheter 14 (or according to arrow F in Figures 7C and 8C). For example, but not in any limiting way, this external shape of flange 21 is rounded, as may be seen in Figures 6A and 6B.

Between distal portion 29 of body 13, or in any event in a position which is closer to that portion than to proximal portion 28, provision is made for two opposing coaxial openings 44 and 45, which are spaced apart, in the wall of body 13. These openings 44 and 45 have an axis M at right angles to that of longitudinal cavity 13A (into which they open) coinciding with the axis W of the safety venous catheter and are capable of containing, in a manner rotating about said axis M, the opposite extremities 12A, 12B of body 12 (or "second body" of cylindrical shape or in any event approximating to a rotation solid, located with a longitudinal axis which can be superimposed upon or substantially superimposed upon aforesaid axis M) inserted into a seat 29K made within body 13 corresponding to distal portion 29. This second body 12 has a through hole 51, having an axis Z at right angles to the M axis, this hole 51 being capable of containing in a through manner cannula 11 when the latter is within catheter 14 or outside protection device 8. In this case hole 51 has an axis Z which substantially coincides with abovementioned axis W and, in particular, contains said catheter within cavity 13A. It will be noted that the terms "substantially coinciding, or "substantially capable of overlapping" mean that the Z axis can precisely overlap and coincide with axis W, but also that such axis Z may be slightly inclined with respect to axis W. This for example may occur because of the fact that the edge of hole 51 bears against cannula 11 after partial initial rotation of body 12 brought about by the magnetic field (as extremity or tip 11A of the cannula emerges from hole 51).

In the same way, these terms indicate that the longitudinal axis of body 12 can precisely overlap axis M or be offset and/or inclined with respect to axis M. In the case of body 12, therefore, for simplicity its longitudinal axis will be indicated as being axis M.

For example, cylindrical body 12 is also a magnet with parts 12N and 12S of opposite polarity or has magnetic portions (12N and 12S) of opposite polarity on its surface 120 in order to act together with parts 13N and 13S of containment body 13. It should be noted that parts having the same polarity in bodies 12 and 13 face each other when cannula 11 is within catheter 14 and can remain in that position (despite their magnetic repulsion) specifically through the presence of cannula 11 within hole 51 in part 12.

Extremities 12A and 12B of cylindrical body or second body 12 are rounded off in order to simplify insertion of that body into seat 29K in containment body 13; collars 55 on surface 120 which are capable of securing moving body 12 within seat 29K of containment body 13 through interference are present close to them. It should be noted that collars 55 are suitably shaped so as to hold body 12 within seat 29K of containment body 13, but allow such body 12 to rotate about its own longitudinal axis M in said seat 29K.

Thus, in accordance with the invention, safety device 8 comprises containment body 13 and second body 12 which is contained within the former and rotates within it, as will be described below.

Thus safety device 8 may be preassembled before insertion into catheter holder 3 and be defined (after pre-assembly) by a single element which can be regularly inserted into cavity 16 of the catheter holder.

In addition to this, the fact that containment body 13 contains second body 12 and determines the zones in which such second body can rotate (defined by the two coaxial openings 44 and 45) ensures that this second body 12 rotates correctly within first body or containment body 13.

It is now assumed that the safety venous catheter in Figures 1-8 is in use. After catheter 14 has been inserted into the patient's body, cannula 11 is extracted from the catheter in the direction of arrow F in Figure 7C.

Cannula 11 slides between containment body 13, which, as shown in the figures, is a solid body with the only through openings 44 and 45 (where second body 12 rotates) and longitudinal cavity 13A. As the tractive force continues, distal extremity 11B of catheter 11 fully penetrates cavity 13A, freeing up hole 51 in moving body 12, and the bevel (or, in general, variation in cross-section) 11C reaches proximal opening 24 of body 13 defined in proximal portion 28 of such body 13.

When bevel 11C reaches portion 28, because cannula 11 has a wider portion at the location of the bevel, the cannula becomes immobilised in containment body 13.

In addition to this, because distal extremity or tip 11B of the cannula projects from hole 51 in second moving cylindrical body 12 and is wholly within longitudinal cavity 13A of the containment body, the second body rotates about its own longitudinal axis M through the effect of the torque generated by the magnetic attraction and repulsion forces between magnetic portion 12N and 12S and parts 13N and 13S of body 13 as mentioned. In particular, second body 12 rotates with its extremities 12A and 12B in openings 44 and 45 of containment body 13.

Rotation takes place within seat 29K around the M axis and in this way hole 51 is positioned with axis Z at least close to being at right angles to axis W of cavity 13; body 12 closes off distal aperture 25 of cavity 13A in containment body 13 and distal extremity 11A of cannula 11 remains trapped within that cavity (the sides of which are closed off).

When bevel (or change in cross-section) 11C present on cannula 11 interferes with the sliding opening for cannula 24, the latter can no longer move back and the traction force (arrow F) exerted by the operator overcomes the interference between arms 34 and body 3 and causes containment body 13 to become detached from catheter holder 3 (Figure 7C). From this time the tip or distal extremity 11B of the cannula (now also separated from catheter holder 3) is protected within body 13 and the risk of accidental punctures for health professionals is minimised.

It will be noted that, from this time on, tip or extremity 11B of cannula 11 is wholly enclosed within containment body 13 (which is wholly closed off from cannula 11 and moving body 12 at its openings 24, 25) and this prevents drops of body fluids present on the tip of the cannula from being projected to the exterior.

The present invention thus improves the types of passive protection devices incorporated in a safety catheter needle, using a magnetic torque to produce a safety system which is substantially without front openings after activation. This torque acts between two bodies, one of which (12) is contained within the other (13), the whole forming a single body 8 from the point of view of assembly and handling at the stage of insertion into catheter holder 3.

The use of magnetic materials for the object described above in comparison with the use of resilient components also makes it possible to obtain a safety system which does not suffer from loss of resilience over time (as in the case of EP0645159). In fact, particularly if constructed of plastics material, resilient protection can suffer relaxation and loss of resilience phenomena (with consequent possible malfunctioning), particularly when stored for long periods under non-optimum conditions. The magnetic materials used in the present invention are conversely such as to possess permanence characteristics in the magnetic field which guarantee a sufficient absence of variation over time, guaranteeing reliability of protection over the entire service life of the device.

Of course the materials used and any coatings used where necessary are such as to ensure the biocompatibility of the device and its conformance with the current legislation and standards applying to medical devices of the type which are the subject matter of the present invention. By way of example, possible magnetic materials which can be used to provide the protection described by the present invention are neodymium-iron-boron, ferrite, samarium-cobalt, aluminium-nickel-cobalt or mixtures thereof.

Possible manufacturing techniques for the invention in question are mechanical machining to remove material, additive technologies, injection moulding of magnetic materials associated with thermoplastic binders, compression moulding and magnetic materials bound with thermohardening epoxy materials, or the sintering of magnetic powders. One example of possible biocompatible components is coating with Teflon, CVD and PVD coatings (for example Parylene, titanium nitrides), sputtering with gold or other bioinert materials, plating with gold or other bioinert materials, or coating with silanes. In general, all biocompatible coatings can be applied where they are suitable for protecting the surface of the magnetic material from possible oxidative effects and where they are suitable for preventing the release or migration of undesired substances, when present, towards the surface of the cannula and catheter holder.

With regard to biocompatibility aspects, in order to support the feasibility of using the present invention reference may be made to the extensive scientific literature which describes the use of permanent magnets in implantable devices. A brief example of relevant scientific literature is represented by the articles:
Physica B 435 (2014) 92-95
J. Prosthet. Dent. 2001; 86: 137-142
Medical Engineering & Physics 34 (2012) 1287-1293
Artificial Organs 2016, 40(3): E12-E24.

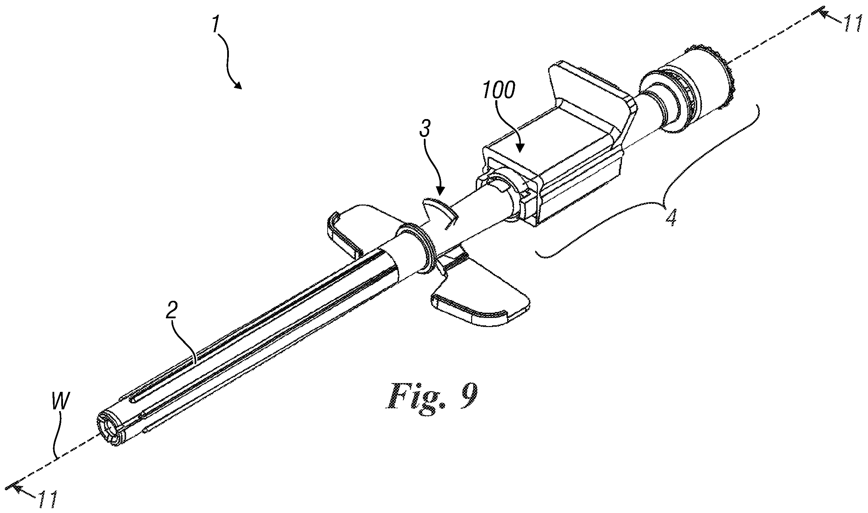

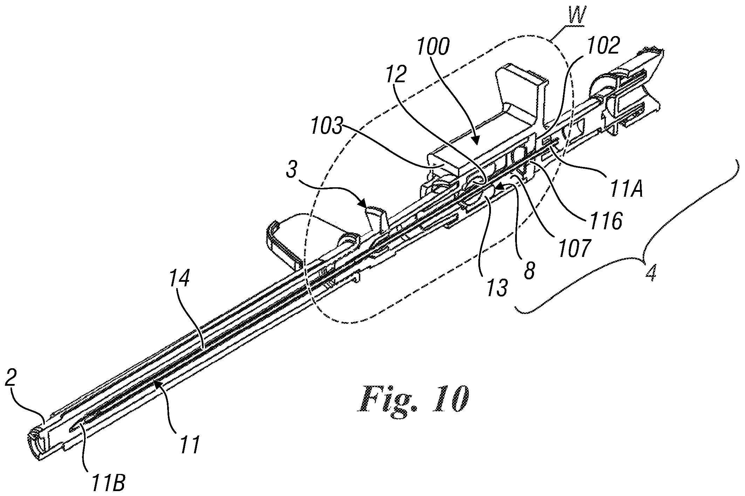

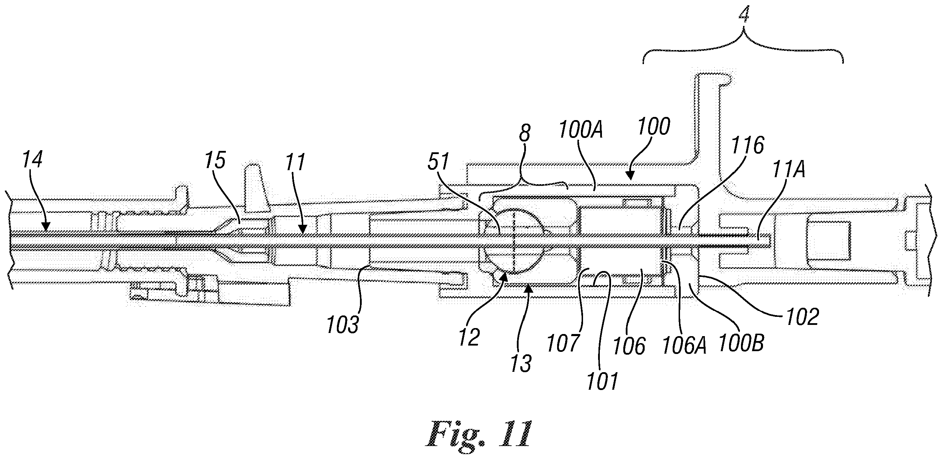

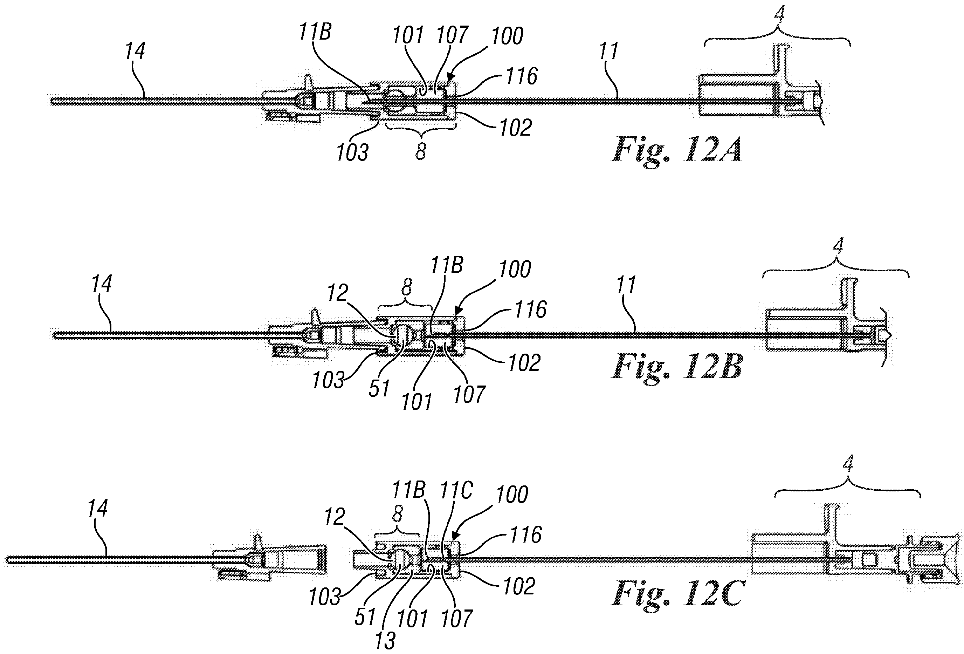

A first variant of the invention is illustrated in Figures 9-12C. In the figures parts corresponding to those in the figures already described are indicated using the same reference numbers.

In the variant in question containment body 13 is inserted within a further housing 100 (comprising at least two parts 100A and 100B attached together in a known way); in turn the entire outer surface of housing 100 is enclosed over and it is removably attached to catheter holder 3. This enclosure 100 (and body 13 containing body 12) define protection device 8.

Containment body 13 has the same characteristics as body 13 in Figures 1-8 (and in particular contains second body 12), but is inserted within a cavity 101 in housing 100 (housing having a proximal extremity 102 and a distal extremity 103). Between containment body 13 and proximal extremity 102 there is an empty space 107 closed off by proximal wall 102 which is pierced at 116 for the passage of cannula 11, but made in such a way as to immobilise said cannula when bevel 11C of the cannula comes close to such wall 102.

In this way, when extracted from the catheter, the distal extremity of cannula 11B is located within space 107 between wall 102 and body 13. This tip or distal extremity 11B is therefore completely inserted within enclosure 100 which protects it and which holds within it any drop of body fluid which might have been released from such extremity 11B when separated from the patient's body.

This solution makes industrial manufacture of protection device 8 easier.

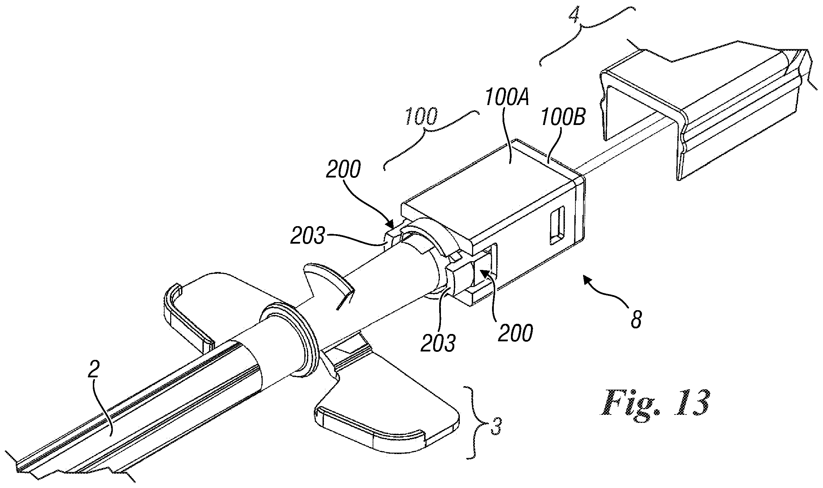

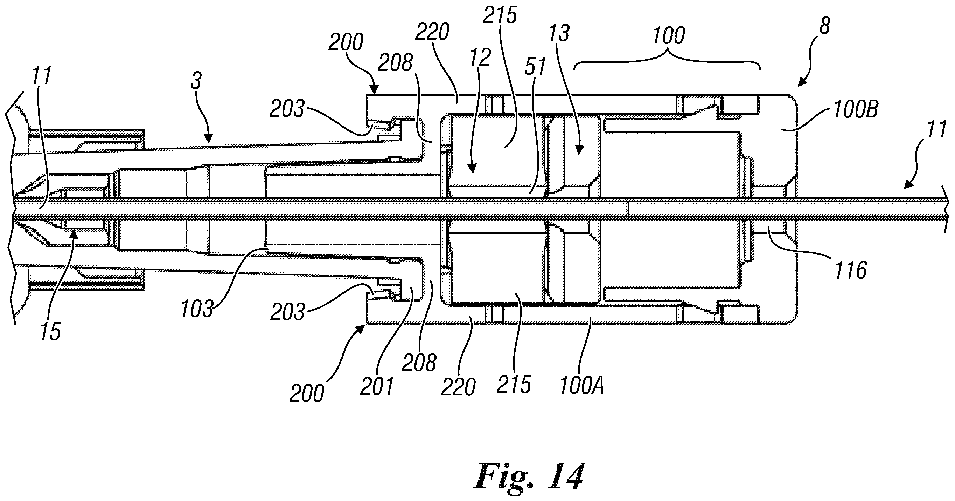

Figures 13-15 show a further variant of the invention which is similar to that in Figures 9-12. In the figures in question parts corresponding to those already described are indicated by the same reference numbers.

In the variant in question housing 100 comprises tabs 200 which can act together with a flange 201 of catheter holder 4. These tabs have an attachment extremity 203 and project at the side of distal extremity 103 of housing 100. The tabs are of one piece with that housing, but are resiliently movable with respect to a weakened zone 208 of housing 100.

Ribs or projections 215 of body 12 act together with these, projecting from two opposite sides 212 and 213 of such body 12.

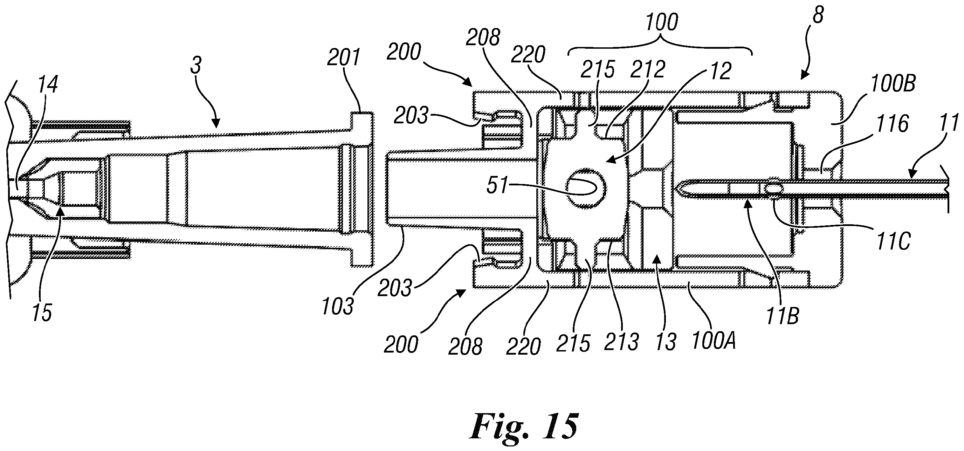

When body 12 (always located within containment body 13) contains cannula 11 passing through it, ribs or projections 215 act together with sides 220 of the tabs and hold it in a position in which it engages flange 201 of the catheter holder. When cannula 11 is extracted from catheter 14, body 12 rotates (Figure 15) about its own longitudinal axis M and these projections 215 move from sides 220 which can thus move back resiliently towards the interior of cavity 101, detaching from catheter holder 3.

Device 8 may be thus separated from catheter holder 3 when extremity 11B of the cannula is in space 107 of housing 100 (Figure 15) through the traction force exerted by an operator on such cannula, the bevel 11C of which acts together with proximal extremity 102 of housing 100. This traction force leads to the abovementioned tabs separating from flange 201 of the catheter holder.

Various embodiments of the invention have been described; yet others are however available to those skilled in the art on the basis of the above and within the scope of the invention defined by the following claims.

## Claims

1. Safety catheter needle comprising a cannula (11) removably inserted in a catheter (14), said cannula (11) being associated with a cannula holder (4), said catheter being associated with a catheter holder (3) which is in turn removably attached to said cannula holder (4), the cannula (11) having one proximal extremity (11A) attached to the cannula holder (4) and a distal extremity (11B) capable of sliding in catheter (14) at the time when the cannula (11) is separated from said catheter (14), close to the distal extremities (11B) of said cannula (11) having a deformed part (11C), being provided with a protection device (8) associated with the catheter holder (3) capable of accommodating said distal extremity (11B) of the cannula (11) after it has been completely detached from the catheter (14), said protection device (8) comprising bodies (12, 13) which act together magnetically and are able to immobilise said distal extremity (11B) of the cannula (11) within said protection device (8), said bodies being a first body (13) and a second body (12) , **characterised in that** both bodies are at least partly magnetised, the first body (13) contains the second body (12), the second body (12) having a transverse longitudinal axis (M) with respect to said first body and being capable of rotating within said first body (13) around such longitudinal axis (M), said second body (12) having a through hole (51) with an axis (Z) which is substantially coaxial with a longitudinal axis (W) of a cavity (13A) in the first body (13) when the distal extremity (11B) of said cannula is outside said protection device (8), said second body (12) being rotated through magnetic repulsion and/or attraction between said first body (13) and about its own longitudinal axis (M) when the distal extremity (11B) of said cannula (11) is within the protection device (8) but outside said through hole (51), the rotated position of said second body (12) around said longitudinal axis (M) and within the aforesaid first body (13) blocking emergence of said distal extremity (11B) of the cannula from said protection device (8).

2. Catheter needle according to claim 1, **characterised in that** the first body (13) has magnetic portions (13N, 13S) facing similar magnetic portions (12N, 12S) when the body (12) has its hole (51) with an axis substantially coaxial with the longitudinal axis (W) of the cavity (13A) of the second body (13).

3. Catheter needle according to claim 1, **characterised in that** said second body (12) is positioned between a seat (29K) made in the first body (13) at one distal extremity (29) of such first body (13), the distal extremity (11B) of the cannula penetrating the protection device (8) from said distal extremity (29) of said first body (13) when it separates from the catheter (14).

4. Catheter needle according to claim 3, **characterised in that** said second body (12) has a shape which at least approximates to a rotational solid of cylindrical shape and has opposite extremities (12A, 12B) which are rotatably inserted into openings (44, 45) provided in the wall of said first body (13), said openings (44, 45) being produced at the seat (29K) of the first body (13), being opposite to each other and spaced apart, and having an axis which can substantially overlap the longitudinal axis (M) of said second body (12), the axis (M) of said openings (44, 45) being at right angles to the longitudinal axis (W) of the cavity (13A) in said first body (13) into which the openings (44, 45) open.

5. Catheter needle according to claim 4, **characterised in that** said second body (12) has close to its two opposite extremities (12A, 12B) and within the first body (13) collars (55) which are capable of preventing such second body (12) from leaving the seat (29K) in the first body.

6. Catheter needle according to claim 1, **characterised in that** said first body (13) has a proximal extremity (28) provided with resiliently deformable means (34) capable of immobilising said first body within the catheter holder (3), but capable of deforming and allowing forced detachment.

7. Catheter needle according to claims 1 and 6, **characterised in that** said cavity (13A) in the first body (13) has within it a collar (31) capable of acting together with the deformed part (11C) of the cannula (11) immobilising it in said cavity (13A) after it has been extracted from the catheter (14).

8. Catheter needle according to claims 1 and 4, **characterised in that** said first body (13) is a cylindrical body which is closed except at its proximal and distal extremities (24, 25) and at the openings (44, 45) in which the extremities (12A, 12B) of the second moving body (12) are positioned.

9. Catheter needle according to claim 1, **characterised in that** said first body (13) rotatably and transversely containing the second body (12) is located within a housing (100) which is removably associated with the catheter holder (3), said housing having a cavity (107) which is capable of containing the distal extremity (11B) of the cannula (11) after extraction from the catheter (14), said first body (13) and said second body (12) preventing such distal extremity (11B) from emerging from said cavity (107).

10. Catheter needle according to claim 9, **characterised in that** said housing (100) is closed except at its proximal (102) and distal (103) extremities in which the cannula (11) can move, said first body (13) being located close to the aforesaid distal extremity (103) .

11. Catheter needle according to claim 10, **characterised in that** said housing (100) comprises attachment means (200) capable of acting together with the catheter holder (3), said attachment means (200) being separable from said catheter holder (3) following rotation of the second body (12) about its own longitudinal axis (M) within the first body (13), separation of said attachment means (200) from the catheter holder (3) permitting complete separation of the protection device (8) of the aforesaid catheter holder.

12. Catheter needle according to claim 1, **characterised in that** said first body (13) and said second body (12) are wholly made of magnetic material.

13. Catheter needle according to claim 1, **characterised in that** said first body (13) and said second body (12) are partly of magnetic material.

14. Catheter needle according to claim 1, **characterised in that** the magnetic parts of said first body (13) and said second body (12) are coated with biocompatible material.

## Patentansprüche

1. Sicherheitskatheternadel, umfassend eine Kanüle (11), die abnehmbar in einen Katheter (14) eingeführt ist, wobei die Kanüle (11) mit einem Kanülenhalter (4) assoziiert ist, wobei der Katheter mit einem Katheterhalter (3) assoziiert ist, der wiederum abnehmbar an dem Kanülenhalter (4) angebracht ist, wobei die Kanüle (11) ein proximales Ende (11A), das am Kanülenhalter (4) befestigt ist, und ein distales Ende (11B), das in den Katheter (14) gleiten kann, wenn die Kanüle (11) vom Katheter (14) getrennt wird, aufweist, in der Nähe des distalen Endes (11B) der Kanüle (11), das ein verformtes Teil (11C) aufweist, mit einer Schutzvorrichtung (8) versehen ist, die mit dem Katheterhalter (3) assoziiert und in der Lage ist, das distale Ende (11B) der Kanüle (11) aufzunehmen, nachdem sie vollständig von dem Katheter (14) gelöst wurde, die Schutzvorrichtung (8) umfassend Körper (12, 13), die magnetisch zusammenwirken und in der Lage sind, das distale Ende (11B) der Kanüle (11) innerhalb der Schutzvorrichtung (8) zu fixieren, wobei die Körper ein erster Körper (13) und ein zweiter Körper (12) sind, **dadurch gekennzeichnet, dass** beide Körper zumindest teilweise magnetisiert sind, wobei der erste Körper (13) den zweiten Körper (12) enthält, wobei der zweite Körper (12) eine transversale Längsachse (M) in Bezug auf den ersten Körper aufweist und in der Lage ist, sich innerhalb des ersten Körpers (13) um diese Längsachse (M) zu drehen, wobei der zweite Körper (12) ein Durchgangsloch (51) mit einer Achse (Z) aufweist, die im Wesentlichen koaxial mit einer Längsachse (W) eines Hohlraums (13A) in dem ersten Körper (13) ist, wenn das distale Ende (11B) der Kanüle außerhalb der Schutzvorrichtung (8) ist, der zweite Körper (12) durch magnetische Abstoßung und/oder Anziehung zwischen dem ersten Körper (13) und um seine eigene Längsachse (M) gedreht wird, wenn das distale Ende (11B) der Kanüle (11) innerhalb der Schutzvorrichtung (8), aber außerhalb des Durchgangslochs (51) ist, wobei die gedrehte Position des zweiten Körpers (12) um die Längsachse (M) und innerhalb des vorgenannten ersten Körpers (13) ein Austreten des distalen Endes (11B) der Kanüle aus der Schutzvorrichtung (8) blockiert.

2. Katheternadel nach Anspruch 1, **dadurch gekennzeichnet, dass** der erste Körper (13) magnetische Abschnitte (13N, 13S) aufweist, die ähnlichen magnetischen Abschnitten (12N, 12S) zugewandt sind, wenn der Körper (12) sein Loch (51) mit einer Achse aufweist, die im Wesentlichen koaxial mit der Längsachse (W) des Hohlraums (13A) des zweiten Körpers (13) ist.

3. Katheternadel nach Anspruch 1, **dadurch gekennzeichnet, dass** der zweite Körper (12) zwischen einem Sitz (29K) positioniert ist, der in dem ersten Körper (13) an einem distalen Ende (29) dieses ersten Körpers (13) gefertigt ist, wobei das distale Ende (11B) der Kanüle die Schutzvorrichtung (8) von dem distalen Ende (29) des ersten Körpers (13) aus durchdringt, wenn sie sich von dem Katheter (14) trennt.

4. Katheternadel nach Anspruch 3, **dadurch gekennzeichnet, dass** der zweite Körper (12) eine Form aufweist, die sich einem Rotationskörper mit zylindrischer Form zumindest annähert und gegenüberliegende Enden (12A, 12B) aufweist, die drehbar in Öffnungen (44, 45) eingesetzt sind, die in der Wand des ersten Körpers (13) bereitgestellt sind, wobei die Öffnungen (44, 45) an dem Sitz (29K) des ersten Körpers (13) hergestellt sind, einander gegenüber und voneinander beabstandet sind und eine Achse aufweisen, die die Längsachse (M) des zweiten Körpers (12) im Wesentlichen überlappen kann, wobei die Achse (M) der Öffnungen (44, 45) rechtwinklig zu der Längsachse (W) des Hohlraums (13A) in dem ersten Körper (13) ist, in den die Öffnungen (44, 45) münden.

5. Katheternadel nach Anspruch 4, **dadurch gekennzeichnet, dass** der zweite Körper (12) in der Nähe seiner zwei gegenüberliegenden Enden (12A, 12B) und innerhalb des ersten Körpers (13) Bunde (55) aufweist, die in der Lage sind, zu verhindern, dass der zweite Körper (12) den Sitz (29K) in dem ersten Körper verlässt.

6. Katheternadel nach Anspruch 1, **dadurch gekennzeichnet, dass** der erste Körper (13) ein proximales Ende (28) aufweist, das mit elastisch verformbaren Mitteln (34) versehen ist, die in der Lage sind, den ersten Körper innerhalb des Katheterhalters (3) zu immobilisieren, die jedoch verformbar sind und ein gewaltsames Lösen ermöglichen.

7. Katheternadel nach den Ansprüchen 1 und 6, **dadurch gekennzeichnet, dass** der Hohlraum (13A) in dem ersten Körper (13) in seinem Inneren einen Bund (31) aufweist, der in der Lage ist, mit dem verformten Teil (11C) der Kanüle (11) zusammenzuwirken und diesen in dem Hohlraum (13A) zu fixieren, nachdem er aus dem Katheter (14) extrahiert worden ist.

8. Katheternadel nach den Ansprüchen 1 und 4, **dadurch gekennzeichnet, dass** der erste Körper (13) ein zylindrischer Körper ist, der außer an seinem proximalen und seinem distalen Ende (24, 25) und an den Öffnungen (44, 45), in denen die Enden (12A, 12B) des zweiten beweglichen Körpers (12) positioniert sind, geschlossen ist.

9. Katheternadel nach Anspruch 1, **dadurch gekennzeichnet, dass** der erste Körper (13), der den zweiten Körper (12) drehbar und quer enthält, sich innerhalb eines Gehäuses (100) befindet, das abnehmbar mit dem Katheterhalter (3) assoziiert ist, wobei das Gehäuse einen Hohlraum (107) aufweist, der in der Lage ist, das distale Ende (11B) der Kanüle (11) nach Extraktion aus dem Katheter (14) aufzunehmen, wobei der erste Körper (13) und der zweite Körper (12) verhindern, dass ein solches distale Ende (11B) aus dem Hohlraum (107) austritt.

10. Katheternadel nach Anspruch 9, **dadurch gekennzeichnet, dass** das Gehäuse (100) mit Ausnahme seines proximalen und seines distalen (103) Endes, in dem sich die Kanüle (11) bewegen kann, geschlossen ist, wobei der erste Körper (13) in der Nähe des vorgenannten distalen Endes (103) angeordnet ist.

11. Katheternadel nach Anspruch 10, **dadurch gekennzeichnet, dass** das Gehäuse (100) Befestigungsmittel (200) umfasst, die in der Lage sind, mit dem Katheterhalter (3) zusammenzuwirken, wobei die Befestigungsmittel (200) nach einer Drehung des zweiten Körpers (12) um seine eigene Längsachse (M) innerhalb des ersten Körpers (13) von dem Katheterhalter (3) trennbar sind, wobei eine Trennung der Befestigungsmittel (200) von dem Katheterhalter (3) eine vollständige Trennung der Schutzvorrichtung (8) des vorgenannten Katheterhalters ermöglicht.

12. Katheternadel nach Anspruch 1, **dadurch gekennzeichnet, dass** der erste Körper (13) und der zweite Körper (12) vollständig aus magnetischem Material gefertigt sind.

13. Katheternadel nach Anspruch 1, **dadurch gekennzeichnet, dass** der erste Körper (13) und der zweite Körper (12) teilweise aus magnetischem Material sind.

14. Katheternadel nach Anspruch 1, **dadurch gekennzeichnet, dass** die magnetischen Teile des ersten Körpers (13) und des zweiten Körpers (12) mit biokompatiblem Material beschichtet sind.

## Revendications

1. Aiguille de cathéter de sécurité comprenant une canule (11) insérée de manière amovible dans un cathéter (14), ladite canule (11) étant associé à un porte-canule (4), ledit cathéter étant associé à un support de cathéter (3) qui est en retour relié de manière amovible audit porte-canule (4), la canule (11) ayant une extrémité proximale (11A) reliée au porte-canule (4) et une extrémité distale (11B) capable de glisser dans le cathéter (14) lorsque la canule (11) est séparée dudit cathéter (14), proche des extrémités distales (11B) de ladite canule (11) ayant une partie déformée (11C), étant fournie avec un dispositif de protection (8) associé au support de cathéter (3) capable d'héberger ladite extrémité distale (11B) de la canule (11) une fois qu'elle a été complètement séparée du cathéter (14), ledit dispositif de protection (8) comprenant des corps (12, 13) qui agissent ensemble magnétiquement et sont capables d'immobiliser ladite extrémité distale (11B) de la canule (11) dans ledit dispositif de protection (8), lesdits corps étant un premier corps (13) et un deuxième corps (12), **caractérisé en ce que** tous les deux corps sont au moins partiellement magnétisés, le premier corps (13) contenant le deuxième corps (12), le deuxième corps (12) ayant un axe longitudinal transversal (M) par rapport audit premier corps et étant capable de tourner à l'intérieur dudit premier corps (13) autour de cet axe longitudinal (M), ledit deuxième corps (12) ayant un trou débouchant (51) avec un axe (Z) qui est sensiblement coaxial avec un axe longitudinal (W) d'une cavité (13A) dans le premier corps (13) lorsque l'extrémité distale (11B) de ladite canule se trouve à l'extérieur dudit dispositif de protection (8), ledit deuxième corps (12) étant mis en rotation par répulsion et/ou attraction magnétique entre ledit premier corps (13) et autour de son propre axe longitudinal (M) lorsque l'extrémité distale (11B) de ladite canule (11) est à l'intérieur du dispositif de protection (8) mais à l'extérieur dudit trou débouchant (5 1), la position en rotation dudit deuxième corps (12) autour dudit axe longitudinal (M) et à l'intérieur du premier corps (13) mentionné ci-dessus bloquant l'émergence de ladite extrémité distale (11B) de la canule dudit dispositif de protection (8).

2. Aiguille de cathéter selon la revendication 1, **caractérisée en ce que** le premier corps (13) a des parties magnétiques (13 N, 13S) faisant face à des parties magnétiques similaires (12N, 12S) lorsque le corps (12) dispose de son trou (51) avec un axe sensiblement coaxial à l'axe longitudinal (W) de la cavité (13A) du deuxième corps (13).

3. Aiguille de cathéter selon la revendication 1, **caractérisée en ce que** ledit deuxième corps (12) est positionné entre un siège (2 9 K) réalisé dans le premier corps (13) à une extrémité distale (29) de ce premier corps (13), l'extrémité distale (11B) de la canule pénétrant le dispositif de protection (8) depuis ladite extrémité distale (29) dudit premier corps (13) lorsqu'elle se sépare du cathéter (14).

4. Aiguille de cathéter selon la revendication 3, **caractérisée en ce que** ledit deuxième corps (12) a une forme qui s'approche au moins d'un solide rotatif de forme cylindrique et a des extrémités opposées (12A, 12B) qui sont insérées de manière rotative dans des ouvertures (44, 45) prévues dans la paroi dudit premier corps (13), lesdites ouvertures (44, 45) étant produites au niveau du siège (29K) du premier corps (13), étant opposées l'une à l'autre et espacées, et ayant un axe qui peut sensiblement chevaucher l'axe longitudinal (M) dudit deuxième corps (12), l'axe (M) desdites ouvertures (44, 45) étant à angle droit par rapport à l'axe longitudinal (W) de la cavité (13A) dans ledit premier corps (13) dans lequel les ouvertures (44, 45) s'ouvrent.

5. Aiguille de cathéter selon la revendication 4, **caractérisée en ce que** ledit deuxième corps (12) a près de ses deux extrémités opposées (12A, 12B) et à l'intérieur du premier corps (13) des colliers (55) capables d'empêcher ce deuxième corps (12) de quitter le siège (29K) dans le premier corps.

6. Aiguille de cathéter selon la revendication 1, **caractérisée en ce que** ledit premier corps (13) a une extrémité proximale (28) munie de moyens élastiquement déformables (34) capables d'immobiliser ledit premier corps à l'intérieur du support de cathéter (3), mais capables de se déformer et de permettre un détachement forcé.

7. Aiguille de cathéter selon les revendications 1 et 6, **caractérisée en ce que** ladite cavité (13A) dans le premier corps (13) comporte à l'intérieur un collier (31) capable d'agir conjointement avec la partie déformée (11C) de la canule (11) en l'immobilisant dans ladite cavité (13A) après son extraction du cathéter (14).

8. Aiguille de cathéter selon les revendications 1 et 4, **caractérisée en ce que** ledit premier corps (13) est un corps cylindrique fermé sauf au niveau de ses extrémités proximale et distale (24, 25) et aux ouvertures (44, 45) dans lesquelles les extrémités (12 A, 12B) du deuxième corps mobile (12) sont positionnées.

9. Aiguille de cathéter selon la revendication 1, **caractérisée en ce que** ledit premier corps (13) contenant de manière rotative et transversale le deuxième corps (12) est situé à l'intérieur d'un boîtier (100) qui est associé de manière amovible au support de cathéter (3), ledit boîtier ayant une cavité (107) capable de contenir l'extrémité distale (11B) de la canule (11) après extraction du cathéter (14), ledit premier corps (13) et ledit deuxième corps (12) évitant que cette extrémité distale (11B) n'émerge de ladite cavité (107) .

10. Aiguille de cathéter selon la revendication 9, **caractérisée en ce que** ledit boîtier (100) est fermé, sauf au niveau de ses extrémités proximale (102) et distale (103) dans lesquelles la canule (11) peut se déplacer, ledit premier corps (13) étant situé près de l'extrémité distale mentionnée ci-dessus (103).

11. Aiguille de cathéter selon la revendication 10, **caractérisée en ce que** ledit boîtier (100) comprend des moyens de fixation (200) capables d'agir conjointement avec le support de cathéter (3), lesdits moyens de fixation (200) étant séparables dudit support de cathéter (3) suite à la rotation du deuxième corps (12) autour de son propre axe longitudinal (M) à l'intérieur du premier corps (13), la séparation desdits moyens de fixation (200) du support de cathéter (3) permettant une séparation complète du dispositif de protection (8) dudit support de cathéter.

12. Aiguille de cathéter selon la revendication 1, **caractérisée en ce que** ledit premier corps (13) et ledit deuxième corps (12) sont entièrement faits en matériau magnétique.

13. Aiguille de cathéter selon la revendication 1, **caractérisée en ce que** ledit premier corps (13) et ledit deuxième corps (12) sont en partie faits en matériau magnétique.

14. Aiguille de cathéter selon la revendication 1, **caractérisée en ce que** les parties magnétiques dudit premier corps (13) et dudit deuxième corps (12) sont enrobés avec du matériau biocompatible.
